# EUROPEAN PATENT SPECIFICATION

(11) **EP 0 339 579 B1**
(45) Date of publication and mention of the grant of the patent: **15.07.1998**
(21) Application number: 89107481.7
(22) Date of filing: 25.04.1989
(51) Int. Cl.: C07D 405/12, C07D 211/22, C07D 405/14, A61K 31/445

(54) **Piperidine compositions and their preparation and use**
Piperidin-Verbindungen und ihre Herstellung und Verwendung
Composés de pipéridine et leur préparation et utilisation

(30) Priority: 28.04.1988 DK 231088
(43) Date of publication of application: 02.11.1989
(73) Proprietor: NOVO NORDISK A/S, 2880 Bagsvaerd (DK)
(72) Inventor: Jakobsen, Palle, DK-3500 Vaerlose (DK); Drejer, Jorgen, DK-3500 Vaerlose (DK)
(74) Representative: Grünecker, Kinkeldey, Stockmair & Schwanhäusser Anwaltssozietät

(56) References cited:
- EP-A- 0 190 496
- EP-A- 0 266 574
- GB-A- 1 422 263

## Description

The present invention relates to therapeutically active piperidine compounds, a method of preparing the same and to pharmaceutical compositions comprising the compounds. The novel compounds are useful in the treatment of anoxia, ischemia, migraine and epilepsy.

It is well known that accumulation of calcium in the brain cells (calcium overload) is seen after periods of uncontrolled hyperactivity in the brain, such as after convulsions, migraine, anoxia and ischemia. As the concentration of calcium in the cells is of vital importance for the regulation of cell function, an uncontrolled high concentration of the cell calcium will lead to, or indirectly cause the symptoms and possibly also the degenerative changes combined with the above diseases.

Therefore calcium overload blockers selective for brain cells will be useful in the treatment of anoxia, ischemia, migraine and epilepsy.

Well known calcium antagonists such as nifedipine, verapamil and diltiazem have activity against pheripheral calcium uptake, e.g. in blood vessels and the heart, however have shown only very low activity against calcium overload in brain cells.

GB-A-1422263 describes 4-phenylpiperidine compounds which are useful as anti-depressants and anti-parkinson agents.

EP-A-0190496 describes piperidine derivatives having activity against disorders relating to damaged gastro-intestinal tissue and to impaired gastro-intestinal motility.

EP-A-0266574 describes piperidine compounds having the following formula 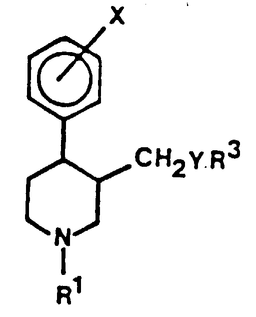 wherein R³
R³ is 3,4-methylenedioxyphenyl, aryl or heteroaryl which are optionally substituted with one or more C₁₋₆-alkyl, C₁₋₆-alkoxy group, C₃₋₈-cycloalkyl, C₃₋₅-alkylene or aralkoxy.
R¹ is straight or branched C₄₋₈-alkyl, C₁₋₈-alkoxy-C₄₋₈-alkyl, C₄₋₇-cycloalkyl, aryloxy-C₃₋₈-alkyl, C₄₋₈-alkenyl or C₄₋₈-cycloalkylalkyl, or R¹ may also be hydrogen or C₁₋₃-alkyl, when R³ is aryl, which is substituted with two or more of C₁₋₆-alkyl, C₁₋₆-alkoxy, C₃₋₈-cycloalkyl, aralkoxy, or with C₃₋₅-alkylene.
X is hydrogen or halogen, and wherein
Y is O or S,
and salts thereof with a pharmaceutically acceptable acid. The compounds are useful in the treatment of anoxia, migraine, ischemia and epilepsy.

Accordingly it is an object of the invention to provide novel compounds having activity against calcium overload in brain cells.

The novel compounds of the invention are piperidine compounds selected from compounds having the general formula I 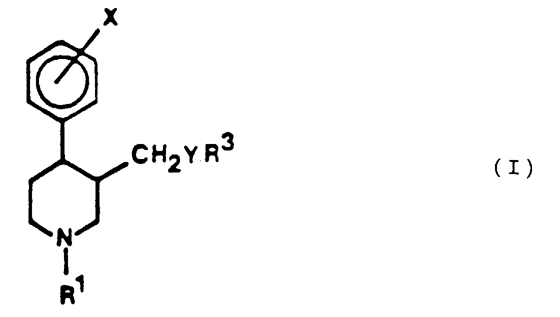 wherein
R³ is is 3,4-methylenedioxyphenyl or phenyl which are optionally substituted with one or more halogen, C₁₋₆-alkoxy, C₁₋₆-alkyl, alkenyl having up to 6 carbon atoms or trifluoromethyl groups,
R¹ is straight or branched C₁₋₈-alkyl unsubstituted or substituted with one or more cyano, hydroxy, amido, halogeno, piperidino, morpholino, thiomorpholino, dioxolanyl, tetrahydrofuranyl, C₁₋₈-alkoxy or C₃₋₈ cycloalkyl groups,
X is hydrogen, halogen, trifluoromethyl, cyano or
C₁₋₈-alkoxy,
Y is O or S,
a salt thereof with a pharmaceutically acceptable acid, and compounds
(+ -)-trans-1-methyl-3-(6-bromo-2-naphthoxymethyl)-4-phenylpiperidine hydrochloride and
(+ -)-trans-3-(4-chloro-1-naphthoxymethyl)-1-methyl-4-phenylpiperidine oxalate
provided that R¹ is not unsubstituted C₁₋₈-alkyl, when R³ is 3,4-methylenedioxyphenyl or phenyl optionally substituted with one or more C₁₋₆-alkyl or C₁₋₆-alkoxy, and at the same time X is hydrogen or halogen, and
further provided that the following compounds are excluded:
cis-3-((4-methoxyphenoxy)-methyl)-1-methyl-4-(4-methoxyphenyl)-piperidine hydrochloride,
cis-3-phenoxymethyl-1-methyl-4-(4-methoxyphenyl)-piperidine hydrochloride,
(-)-cis-3-((4-methoxyphenoxy)-methyl)-1-trifluoroethyl-4-phenylpiperidine dec. hydrochloride,
(+)-cis-3-((4-methoxyphenoxy)-methyl)-1-trifluoroethyl-4-phenylpiperidine dec. hydrochloride,
(+-)-cis-3-((4-methoxyphenoxy)methyl)-1-trifluoroethyl-4-phenylpiperidine hydrobromide,
(-)trans-4-(4'-fluorophenyl)-1-methyl-3-(3'-trifluoromethylphenoxymethyl)-piperidine,
(+)trans-3-(4'-fluorophenoxymethyl)-4-(4'-fluorophenyl)-1-methylpiperidine and α-3-((4-chlorophenoxy)-methyl)-1-methyl-4-phenyl-piperidine hydrobromide.

Preferred compounds of formula I are compounds wherein, R³ is 3,4-methylenedioxyphenyl, optionally substituted with halogen or C₁₋₆-alkoxy, and/or R¹ is straight or branched C₁₋₈-alkyl, and/or X is hydrogen, halogen, trifluoromethyl or C₁₋₆-alkoxy.

Examples of such salts include inorganic and organic acid addition salts such as hydrochloride, hydrobromide, sulphate, phosphate, acetate, fumarate, maleate, citrate, lactate, tartrate, oxalate, or similar pharmaceutically-acceptable inorganic or organic acid addition salts.

The invention also relates to a method of preparing the above mentioned compounds.These methods comprise
a) reacting a compound having the general formula II 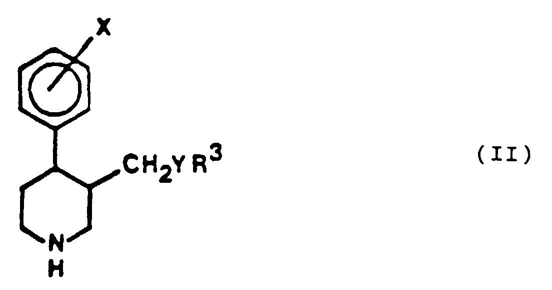 wherein R³, X and Y have the meanings defined above, with a compound having the general formula R¹-Z , wherein Z is a leaving group such as halogen and R¹ has the meaning defined above, or
b) reacting a compound having the general formula III 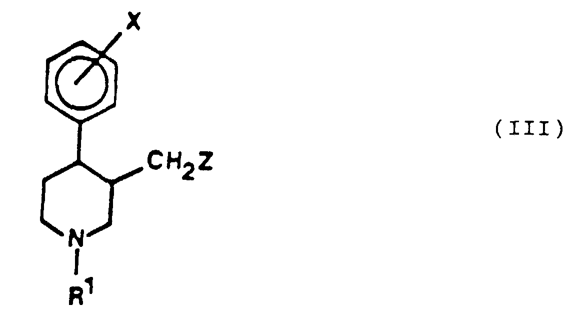 wherein R¹ and X have the meanings defined above, and Z is a leaving group, with a compound having the the general formula R³-YH, wherein Y is O or S and R³ has the meaning defined above, or
c) reacting a compound having the general formula I, 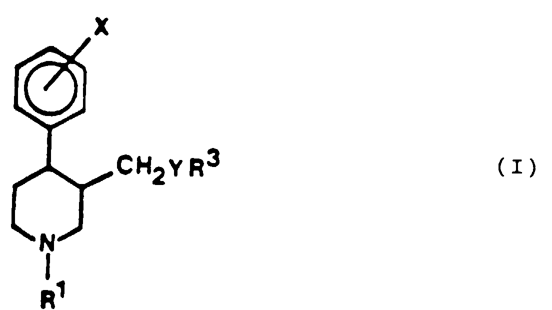 wherein X, R¹, R³ and Y have the meanings defined above, with bromine, and optionally thereafter forming a salt with a pharmaceutically acceptable acid.

The invention also relates to the use of a compound selected from compounds having the general formula I 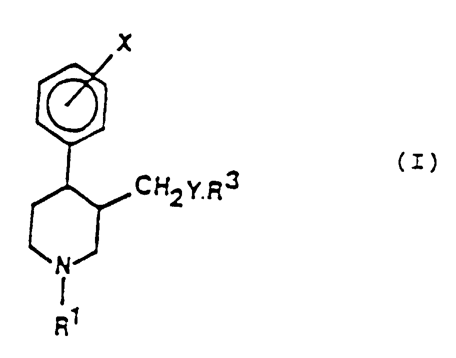 wherein
R³ is 3,4-methylenedioxyphenyl or phenyl which are optionally substituted with one or more halogen, C₁₋₆-alkoxy, C₁₋₆-alkyl, alkenyl having up to 6 carbon atoms or trifluoromethyl groups,
R¹ is straight or branched C₁₋₈-alkyl unsubstituted or substituted with one or more cyano, hydroxy, amido, halogeno, piperidino, morpholino, thiomorpholino, dioxolanyl, tetrahydrofuranyl, C₁₋₈-alkoxy or C₃₋₈ cycloalkyl groups,
X is hydrogen, halogen, trifluoromethyl, cyano or C₁₋₈-alkoxy,
Y is O or S
a salt thereof with a pharmaceutically acceptable acid, and compounds
(+ -)-trans-1-methyl-3-(6-bromo-2-naphthoxymethyl)-4-phenylpiperidine hydrochloride and
(+ -)-trans-3-(4-chloro-1-naphthoxymethyl)-1-methyl-4-phenylpiperidine oxalate
provided that R¹ is not unsubstituted C₁₋₈-alkyl, when R³ is 3,4-methylenedioxyphenyl or phenyl optionally substituted with one or more C₁₋₆-alkyl or C₁₋₆-alkoxy, and at the same time X is hydrogen or halogen,
for the preparation of a medicament useful in the treatment of calcium overload in brain cells of mammals.

The pharmacological properties of the compounds of the invention can be illustrated by determining their capability to inhibit calcium uptake into brain synaptosomes.

### PRINCIPLE

Depolarization of neuronal membranes leads to an opening of so-called 'voltage operated calcium channels' (VOC) in the membranes which allows a massive influx of calcium from the extracellular space. A crude synaptosomal preparation (so-called P₂ fraction) contains small vesicles surrounded by neuronal membrane and it is possible in such a preparation to study a depolarization-induced opening of VOC. In the present model ⁴⁵Ca influx is induced in the synaptosomes by depolarization with elevated potassium concentrations, and the effect of test substances on this stimulated uptake is studied (Nachshen, D.A. and Blaustein, M.P., Mol. Pharmcol., 16, 579 (1979)).

### ASSAY

A male Wistar rat is decapitated and the cerebral cortex removed and homogenized in 10 ml of ice-cold 0.32 M sucrose using a glass homogenizer with a teflon pestle. All subsequent steps for isolation of synaptosomes are done at 0-4°C. The homogenate is centrifuged at 1000 x g for 10 min and the resulting supernatant is re-centrifuged at 18000 x g for 20 min. This pellet (P₂) is resuspended in 0.32 M sucrose (5 ml per g of original tissue) with a teflon pestle.

Aliquots (0.050 ml) of this crude synaptosomal suspension are added to glass tubes containing 0.625 ml of NaCl buffer (136 mM NaCl, 4 mM KCl, 0.35 mM CaCl₂, 1.2 mM MgCl₂, 20 mM Tris HCl, 12 mM glucose, pH 7.4) and 0.025 ml of various drug solutions in 48% Ethanol. The tubes are pre-incubated for 30 min on ice and then for 6 min at 37°C in a water bath.

The uptake is immediately initiated by adding 0.4 ml of ⁴⁵CaCl₂ (specific activity = 29-39 Ci/g; 0.5 Ci/assay), in 145 mM NaCl for non-depolarized samples and in 145 mM KCl for depolarized samples. The incubation is continued for 15 s.

The uptake is terminated by rapid filtration through GF-C glass fiber filters which are washed three times with 5 ml of a cold solution containing 145 mM KCl, 7 mM EGTA and 20 mM Tris HCl, pH 7.4. The amount of radioactivity on the filter disc is determined by liquid scintillation spectrometry.

### TEST PROCEDURE

Test substances are dissolved in 10 ml of 48% ethanol at a concentration of 0.44 mg/ml. Dilutions are made in 48% ethanol. Experiments are performed in quadruplicate. Controls for depolarized and nondepolarized samples are included in the assay and test substances are only tested in depolarized samples.

### RESULTS

Test values are given as MEC (minimal effective concentration, µg/ml), which inhibit stimulated uptake of ⁴⁵Ca significant different (P < 0.05, Student's t-test) from control.

Test results obtained by testing some compounds of the present invention are given in the following table 1.

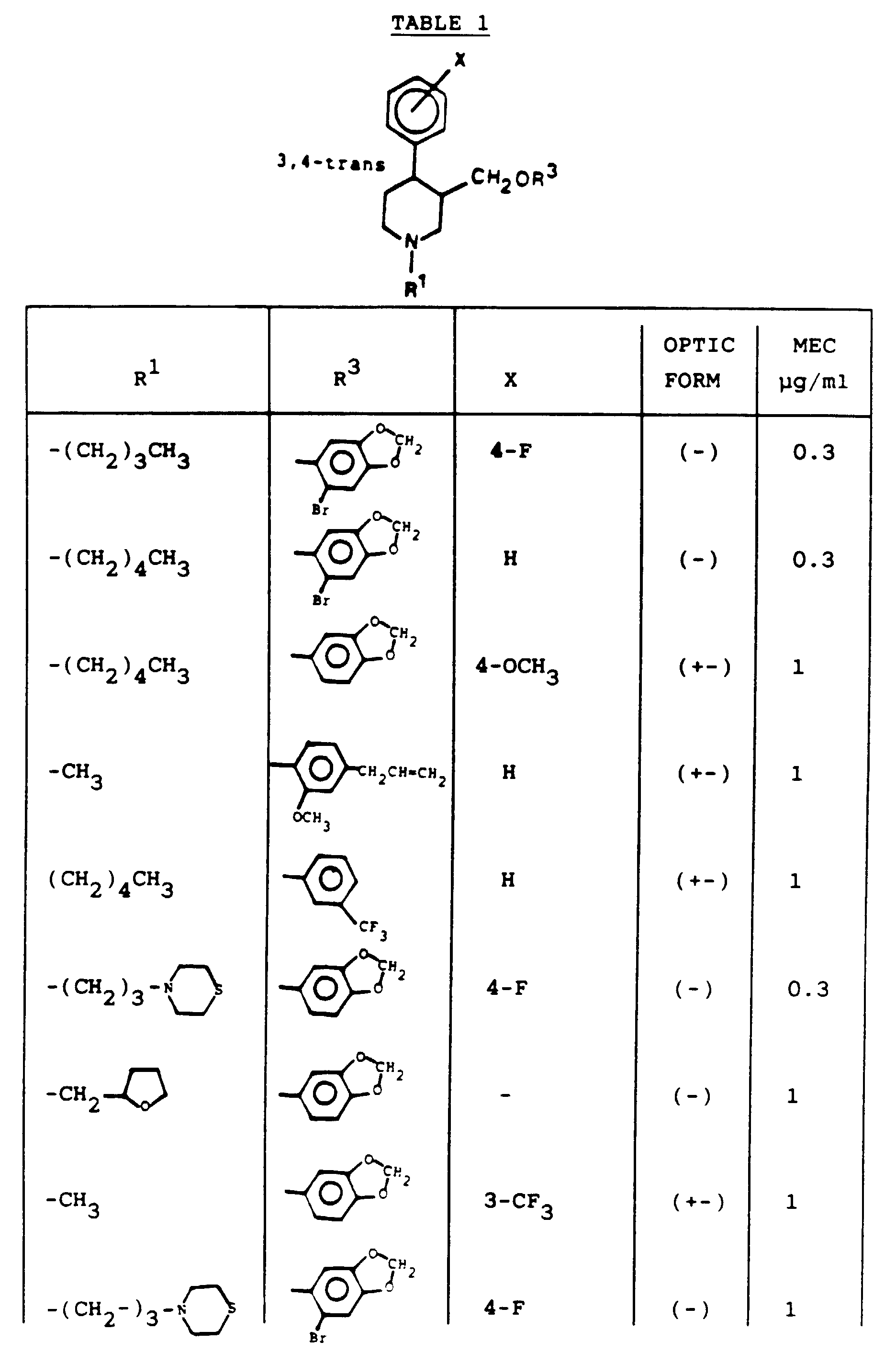

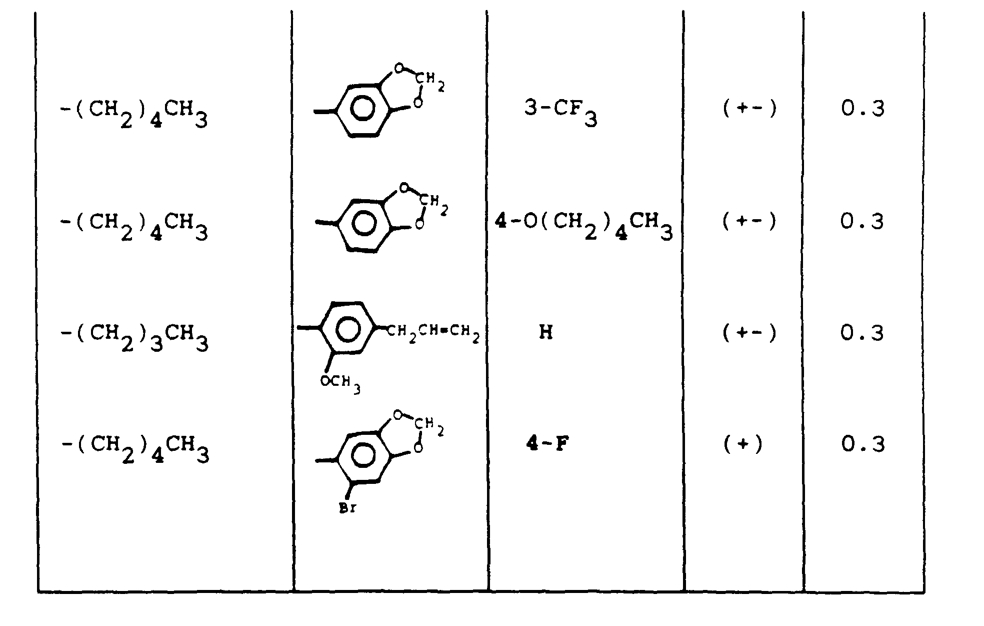

The compounds of the invention, together with a conventional adjuvant, carrier, or diluent, and if desired in the form of a pharmaceutically-acceptable acid addition salt thereof, may be placed into the form of pharmaceutical compositions and unit dosages thereof. In such forms they may be employed as solids, such as tablets or filled capsules, or liquids, such as solutions, suspensions, emulsions, elixirs, or capsules filled with the same, all for oral use; in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective calcium overload blocking amount of the active ingredient commensurate with the intended daily dosage range to be employed. Tablets containing ten (10) milligrams of active ingredient or, more broadly, ten (10) to hundred (100) milligrams, per tablet, are accordingly suitable representative unit dosage forms.

The compounds of this invention can thus be used for the formulation of pharmaceutical preparations, e.g. for oral and parenteral administration to mammals including humans, in accordance with conventional methods of galenic pharmacy.

Conventional excipients are such pharmaceutically acceptable organic or inorganic carrier substances suitable for parenteral or enteral application which do not deleteriously react with the active compounds.

Examples of such carriers are water, salt solutions, alcohols, polyethylene glycols, polyhydroxyethoxylated castor oil, gelatine, lactose, amylose, magnesium stearate, talc, silicic acid, fatty acid monoglycerides and diglycerides, pentaerythritol fatty acid esters, hydroxymethylcellulose and polyvinylpyrrolidone.

The pharmaceutical preparations can be sterilized and mixed, if desired, with auxiliary agents, emulsifiers, salt for influencing osmotic pressure, buffers and/or coloring substances and the like, which do not deleteriously react with the active compounds.

For parenteral application, particularly suitable are injectable solutions or suspensions, preferably aqueous solutions with the active compound dissolved in polyhydroxylated castor oil.

Ampoules are convenient unit dosage forms.

Tablets,dragees, or capsules having talc and/or a carbohydrate carrier or binder or the like, the carrier preferably being lactose and/or corn starch and/or potato starch, are particularly suitable for oral application. A syrup, elixir or the like can be used in cases where a sweetened vehicle can be employed.

Generally, the compounds of this invention are dispensed in unit form comprising 0.05-100 mg in a pharmaceutically acceptable carrier per unit dosage.

The dosage of the compounds according to this invention is 0.1-300 mg/day, preferably 10-100 mg/day, when administered to patients, e.g. humans, as a drug.

A typical tablet which may be prepared by conventional tabletting techniques contains:

| | |
|---|---|
| Active compound | 5.0 mg |
| Lactosum | 67.8 mg Ph.Eur. |
| Avicel™ | 31.4 mg |
| Amberlite™IRP 88 | 1.0 mg |
| Magnesii stearas | 0.25 mg Ph.Eur |

Due to the high calcium overload blocking activity , the compounds of the invention are extremely useful in the treatment symptoms related to an accumulation of calcium in brain cells of mammals , when administered in an amount effective for blocking calcium overload in brain cells . The important calcium overload blocking activity of compounds of the invention includes both activity against anoxia, ischemia, migraine and epilepsy . The compounds of the invention may accordingly be administered to a subject, e.g., a living animal body, including a human, in need of a calcium overload blocker , and if desired in the form of a pharmaceutically-acceptable acid addition salt thereof (such as the hydrobromide, hydrochloride, or sulfate, in any event prepared in the usual or conventional manner, e.g., evaporation to dryness of the free base in solution together with the acid), ordinarily concurrently, simultaneously, or together with a pharmaceutically-acceptable carrier or diluent, especially and preferably in the form of a pharmaceutical composition thereof, whether by oral, rectal, or parenteral (including subcutaneous) route, in an effective calcium overload blocking amount, and in any event an amount which is effective for the treatment of anoxia, ischemia, migraine or epilepsy, traumatic head injury and neurodegenerative diseases due to their calcium overload blocking activity. Suitable dosage ranges are 1-200 milligrams daily, 10-100 milligrams daily, and especially 30-70 milligrams daily, depending as usual upon the exact mode of administration, form in which administered, the indication toward which the administration is directed, the subject involved and the body weight of the subject involved, and the preference and experience of the physician or veterinarian in charge.

The invention will now be described in further detail with reference to the following examples:

### EXAMPLE 1

### (-)-trans-1-(2-cyanoethyl)-4-(4-fluorophenyl)-3-(3,4-methylenedioxyphenoxymethyl)-piperidine hydrochloride

1 g of (-)-trans-4-(4-fluorophenyl)-3-(3,4-methylene dioxyphenoxymethyl)-piperidine hydrochloride in 50 ml 99.9% ethanol was mixed with 3-bromopropionitrile (7 ml) and 2 g potassium carbonate. The mixture was refluxed for 70 h. After cooling 25 ml acetone and 25 ml diethylether were added, the precipitate filtered off, and the filtrate evaporated in vacuo. The residue was extracted with 1 N NaOH/ether, the ether layer dried (MgSO₄) and evaporated to dryness. The residue was dissolved in acetone and excess conc. HCl was added. Subsequent evaporation gave a hard glass, which was purified on a silica gel column using 99.5% ethanol as eluent. The title compound was isolated, and its structure confirmed by the IR and NMR data. M.p. 156°C.

The following compounds were prepared in the same manner from (-)-trans-4-(4-fluorophenyl)-3-(3,4-methylenedioxyphenoxymethyl)piperidine hydrochloride and the relevant halogeno compound (the actual halogen given). Oxalates were prepared from the free base by mixing equimolar amounts of amine and oxalic acid (anhydrous) in acetone solution, which caused precipitation of the oxalate after few min at RT or in the fridge:

(-)-trans-1-(3-(4,4-dimethyl-1-piperidyl)-propyl)-4-(4-fluorophenyl)-3-(3,4-methylenedioxyphenoxymethyl)-piperidine dihydrochloride, from equimolar amounts of the "piperidine" and the chloro compound. Reflux time 190 h, m.p. 267°C.
(-)-trans-4-(4-fluorophenyl)-3-(3,4-methylenedioxyphenoxymethyl)-1-(3-(2-methyl-1-piperidyl)propyl)-piperidine dihydrochloride, from equimolar amounts of "piperidine" and the chloro compound by reflux for 3 h, a few crystals of I₂ added. M.p. 250°C.
(-)-trans(2-ethoxycarbonylethyl)-4-(4-fluorophenyl)-3-(3,4-methylene dioxyphenoxymethyl)piperidine oxalate, from the bromo compound, reflux time 2 h, m.p. 51°C, purified by column chromatography on silicagel using CH₂Cl₂/CH₂OH 9:1 as eluent.
(-)-trans-4-(4-fluorophenyl)-3-(3,4-methylenedioxyphenoxymethyl)-1-(3-thiomorpholinylpropyl)-piperidine dihydrochloride, from equimolar amounts of "piperidine" and the chloro compound, a few crystals I₂ added, reflux time 3 h, m.p. 267°C.
(-)-trans-1-carbamoylmethyl-4-(4-fluorophenyl)-3-(3,4-methylenedioxyphenoxymethyl)-piperidine hydrochloride, from the iodo compound, reflux for 2 h, m.p. 104°C.
(-)-trans-4-(4-fluorophenyl)-3-(3,4-methylenedioxyphenoxymethyl)-1-(3-morpholinopropyl)-piperidine dihydrochloride, from equimolar amounts of "piperidine" and the chloro compound, a few crystals of iodine added, reflux for 30 h, m.p. 108°C.
(-)-trans-1-(4-cyanobutyl)-4-(4-fluorophenyl)-3-(3,4-methylenedioxyphenoxymethyl)-piperidine oxalate, from the bromo compound by addition of a few iodine crystals, and reflux for 1 h. The free bases was purified on a silicagel column using CH₂Cl₂/CH₃OH 9:1 as eluent, m.p. 89°C.
(-)-trans-1-(1,3-dioxolyl-2-methyl)-4-(4-fluorophenyl)-3-(3,4-methylenedioxyphenoxymethyl)-piperidine oxalate, from the bromo compound, addition of one iodine crystal, reflux for 120 h, m.p. 53°C.
(-)-trans-4-(4-fluorophenyl)-1-tetrahydrofurfuryl-3-(3,4-methylenedioxyphenoxymethyl)-piperidine )-piperidine oxalate, from the bromo compound, reflux time 7 h, purified on silicagel column, eluent CH₂Cl₂/CH₃OH 9:1, hard glass. Identified by NMR and MS data. MS (m/e, % of base peak): 413,5; 343,38; 342,100; 204,25; 137,28; 109,38; 83,42; 58,100; 57,55.
(-)-trans-4-(4-fluorophenyl)-1-(6-hydroxyhexyl)-3-(3,4-methylenedioxyphenoxymethyl)-piperidine oxalate, from the chloro compound, addition of a few crystals of iodine, reflux for 24 h, purified by column chromatography on silicagel CH₂Cl₂/CH₃OH 9:1 as eluent hard glass. Identified by IR, NMR and MS-data. MS (m/e, % og base peak): 429,3; 343,15; 342,55; 204,10; 171,10; 137,12; 109,15; 58,100.
(-)-trans-4-(4-fluorophenyl)-1-(3-hydroxypropyl)-3-(3,4-methylenedioxyphenoxymethyl)-piperidine oxalate, from the bromo compound, reflux 7 h, isolated as a hard glass, identified by IR and NMR.

### EXAMPLE 2

### (+-)-trans-1-methyl-3-(6-bromo-2-naphthoxymethyl)-4-phenylpiperidine hydrochloride

6-bromo-2-naphthol (2.45 g) was dissolved in MIBC (40 ml). NaOH (0.52 g) was added, and the mixture was stirred for ½ h. (+-)-trans-1-methyl-4-phenyl-3-phenyl-sulfonyloxymethylpiperidine (3.5 g) dissolved in MIBC (50 ml) was added to the "phenolate" solution, heating to 110°C for 6 h. The reaction mixture was evaporated to dryness and the residue extracted with OH/ether. The ether layer was dried (Na₂SO₄) filtered and evaporated to dryness. The crude product was purified on silicagel, petrolether/CH₃OH 1:1 as eluent. The purified product was dissolved in ether and precipitated with excess conc. HCl-solution. Reprecipitation from acetone/ether gave 0.7 g compound, m.p.225°C.

In the same manner were prepared the following compounds from (+-)-trans-1-methyl-4-phenyl-3-phenylsulfonyloxymethylpiperidine and the appropriate substituted phenol or naphthol. Oxalates were prepared by mixing equimolar amounts of "piperidine base" and anhydrous oxalic acid in acetone solution.
(+-)-trans-1-methyl-3-(3-trifluoromethylphenoxymethyl-4-phenylpiperidine oxalate. Heating at 130°C until the sulfoester had reacted as seen by TLC. M.p. 92°C.
(+-)-trans-3-(4-chloro-1-naphthoxymethyl)-1-methyl-4-phenylpiperidine oxalate. Heating to 110°C for 14 h. M.p. 88°C.
(+-)-trans-3-(4-allyl-2-methoxyphenoxymethyl)-4-phenylpiperidine oxalate. Reaction time 40 h at 110°C. M.p. 137°C.
(+-)-trans-1-methyl-3-(3-phenoxyphenoxymethyl)-4-phenylpiperidine oxalate. M.p. 166°C.
(+-)-trans-3-(2-cyanophenoxymethyl)-1-methyl-4-phenylpiperidine oxalate. M.p. 108-110°C.

### EXAMPLE 3

### (+-)-trans-3-(3-trifluoromethylphenoxymethyl)-4-phenylpiperidine hydrochloride

was prepared by means of alpha-chloroethyl chloroformate using the method described in J. Org. Chem. 49 (1984) 2081 (R.A. Olofson, J.T. Martz, J.P. Senel, M. Piteau and T. Malfroot). Na-dried toluene was used as solvent instead of 1,2- dichloroethane in the primary reaction. M.p. 171°C.

The following compounds were prepared in exactly the same manner by N-dealkylation of the corresponding N-methyl compound.

(+-)-trans-4-(4-methoxyphenyl)-3-(3,4-methylenedioxyphenoxymethyl)-piperidine. The hydrochloride was extracted with NaOH/ether, the above mentioned compound was precipitated from acetone/ether, m.p. 184°C.

(+-)-trans-3-(4-allyl-2-methoxyphenoxymethyl)-4-phenylpiperidine oxalate. The hydrochloride was extracted with OH⁻/ether, the ether phase evaporated to dryness, and the residue dissolved in acetone and precipitated with an equimolar amount of anhydrous oxalic acid in acetone solution, m.p. 101°C.

(+-)-trans-3-(3-phenoxyphenoxymethyl)-4-phenylpiperidine oxalate. The hydrochloride was extracted with OH⁻/ether, the ether phase evaporated to dryness, and the residue dissolved in acetone and precipitated by means of an equimolar amount of anhydrous oxalic acid in acetone solution. M.p. 138-142°C.

### EXAMPLE 4

The following compounds were prepared using the alkylation method described in example 1.
(+-)-trans-3-(3-trifluoromethylphenoxymethyi)-1-pentyl-4-phenylpiperidine oxalate, from (+-)-trans-3-(3-trifluoromethylphenoxymethyl)-4-phenylpiperidine and pentyl bromide by reflux for 10 h. M.p. 130°C.
(+-)-trans-4-(4-methoxyphenyl)-3-(3,4-methylenedioxyphenoxymethyl)-1-pentylpiperidine oxalate, from the corresponding unsubstituted piperidine and 1-bromopentane by reflux for 12 h, m.p. 213°C.
(+-)-trans-3-(4-allyl-2-methoxyphenoxymethyl)-1-pentyl-4-phenylpiperidine oxalate, by reflux of the corresponding unsubstituted piperidine with pentyl bromide for 16 h, m.p. 116°C.
(+-)-trans-3-(3,4-methylenedioxyphenoxymethyl)-1-pentyl-4-(3-trifluoromethylphenyl)-piperidine hydrochloride from the corresponding unsubstituted piperidine by reflux for 1 h with pentylbromide. M.p. 166.6°C.
(+-)-trans-1-pentyl-3-(3-phenoxyphenoxymethyl)-4-phenylpiperidine oxalate from the corresponding unsubstituted piperidine and pentylbromide by reflux for 2 h. M.p. 77°C.
(+-)-3-(4-allyl-2-methoxyphenoxymethyl)-1-pentyl-4-(3-trifluoromethylphenyl)-piperidine oxalate, prepared from 1-bromopentane and 3-(4-allyl-2-methoxyphenoxymethyl)-4-(3-trifluoromethylphenyl)piperidine by reflux for 10 h. M.p. 130.4°C.
(+-)-3-(4-allyl-2-methoxyphenoxymethyl-1-pentyl-4-(4-trifluoromethylphenyl)-piperidine oxalate, prepared from the corresponding unsubstituted piperidine as the oxalate and pentylbromide, purified on silicagel column CH₂Cl₂/CH₃OH 9/1 as eluent. M.p. 141.2°C.
(+-)-trans-3-(4-allyl-2-methoxyphenoxymethyl)-1-butyl-4-phenylpiperidine oxalate. Preparation from 1-bromobutane and the unsubstituted piperidine by reflux for 5.5 h. M.p. 74.9°C.
(+-)-trans-3-(4-allyl-2-methoxyphenoxymethyl)-1-cyclopropylmethyl)-4-phenylpiperidine oxalate, from cyclopropylmethylbromide and unsubstituted piperidine by reflux for 2 h. M.p. 80.1°C.
(+-)-trans-3-(4-allyl-2-methoxyphenoxymethyl)-4-phenyl-1-propylpiperidine oxalate, from 1-bromopropane and unsubstituted piperidine by reflux for 6 h. M.p. 81°C.
(+-)-trans-3-(4-allyl-2-methoxyphenoxymethyl)-1-hexyl-4-phenylpiperididne oxalate, from the corresponding unsubstituted piperidine and 1-bromohexane by reflux for 144 h. M.p. 114°C.

### EXAMPLE 5

### (+-)-trans-4-(4-methoxyphenyl)-1-methyl-3-(3,4-methylenedioxyphenoxymethyl)-piperidine, hydrochloride

(+-)-trans-3-methoxycarbonyl-4-(4-methoxyphenyl)-1-methylpiperidine was prepared from arecoline and 4-bromoanisole as described by Plati et. al. (J. Org. Chem. 22 (1957) 261).

9.6 g of this compound was reduced with LiAlH₄ (2.8 g) in dry ether (150 ml), by reflux for 6 h, giving (+-)-trans-3-hydroxymethyl-4-(4-methoxyphenyl)-1-methylpiperidine (6.5 g) as an oil when the normal rinse-up procedure was used.

The crude product was dissolved in toluene (300 ml) triethylamine (7.7 ml) was added, and after stirring for ½ h benzenesulphonyl chloride (4.3 ml) was added, and the mixture stirred at R.T. for 5 h.

The toluene phase was washed with H₂O, dried over MgSO₄, filtered and evaporated to dryness resulting in 7.9 g of (+-)-trans-4-(4-methoxyphenyl)-1-methyl-3-phenylsulfonyloxymethylpiperidine as a yellow oil.

4.1 g of this oil dissolved in MIBC (200 ml) was added to a solution of sesamole (1.7 g) and NaOH (0.5 g) in MIBC (200 ml). The mixture was stirred at reflux temp. for 1.5 h. Subsequently the mixture was extracted with H₂O. The MIBC-phase was isolated and evaporated to dryness.

The resulting mass was extracted from aqueous NaOH/ether, the ether layer was isolated, dried over MgSO₄ and evaporated to dryness. The resulting oil was dissolved in acetone and precipitated as its hydrochloride salt by addition of excess conc. HCl-solution.

Yield 1.7 g of (+-)-trans-4-(4-methoxyphenyl)-1-methyl-3-(3,4-methylenedioxyphenoxymethyl)-piperidine, hydrochloride. M.p. 212.2°C. The identity was confirmed by the IR, NMR and MS-data.

(+-)-trans-1-methyl-3-(3,4-methylenedioxyphenoxymethyl)-4-(3-trifluoromethylphenyl)piperidine was prepared using the same reaction sequence starting from arecoline and 1-bromo-3-trifluoromethyl-benzene. M.p. 93.6°C.

### EXAMPLE 6

### (-)-trans-3-(2-bromo-4,5-methylenedioxyphenoxymethyl)-1-butyl-4-(4-fluorophenyl)-piperidine hydrochloride

(-)-trans-1-butyl-4-(4-fluorophenyl)-3-(3,4-methylenedioxyphenoxymethyl)-piperidine hydrochloride (1 g) was dissolved in CH₂Cl₂ (50 ml). Bromine (0.124 ml) was added dropwise at R.T. After stirring for 2 h aqueous NaOH was added, and the CH₂Cl₂ layer was isolated, dried over Na₂SO₄, filtered and evaporated to dryness. The residue was dissolved in acetone, excess conc. HCl was added, and the above mentioned bromo compound was precipitated by addition of ether. M.p. 116°C.

In exactly the same manner the following compounds were prepared from the corresponding unbrominated compounds.
(-)-trans-3-(2-bromo-4,5-methylenedioxyphenoxymethyl)-1-pentyl-4-phenylpiperidine hydrochloride, m.p. 156°C.
(-)-trans-3-(2-bromo-4,5-methylenedioxyphenoxymethyl)-4-(4-fluorophenyl)-1-pentylpiperidine hydrochloride. M.p. 105°C.
(-)-trans-3-(2-bromo-4,5-methylenedioxyphenoxymethyl)-4-(4-fluorophenyl)-1-(2-methoxyethyl)-piperidine hydrochloride. M.p. 65°C (hard glass).
(-)-trans-3-(2-bromo-4,5-methylenedioxyphenoxymethyl)-1-cyclopropylmethyl-4-(4-fluorophenyl)-piperidine hydrochloride. M.p. 60°C (hard glass)
(-)-trans-3-(2-bromo-4,5-methylenedioxyphenoxymethyl)-1-(2,3-dibromopropyl)-4-(4-fluorophenyl)-piperidine hydrochloride. The crude product was purified on silicagel using CH₂Cl₂, CH₃OH 9/1 as eluent. M.p. 108°C.
(-)-trans-3-(2-bromo-4,5-methylenedioxyphenoxymethyl)-4-(4-fluorophenyl)-1-(3-thiomorpholinopropyl)piperidine dihydrochloride. The crude product was purified on silicagel using CH₂Cl₂/CH₃OH 9/1 as eluent. M.p. 241°C.
(+)-trans-3-(2-bromo-4,5-methylenedioxyphenoxymethyl)-4-(4-fluorophenyl)-1-pentylpiperidine hydrochloride. M.p. 112.3-113.3°C.

### EXAMPLE 7

### (+-)-1-methyl-3-(3,4-methylenedioxyphenoxymethyl)-4-(3-trifluoromethylphenyl)-piperidine

3-methoxycarbonyl-1-methyl-4-(3-trifluoromethylphenyl)piperidine was prepared as the cis/trans mixture from arecoline and 3-bromo-trifluoromethylbenzene as described (J.Org.Chem. 22 (1957) 261). The product was purified by vacuum distillation. B.p. 90-100°C/0.7 mmHg.

19.2 g of this compound was reduced by means of LiAlH₄ (4.85 g) in dry ether (325 ml) in an N₂-atmosphere by reflux for 4 h. After the normal rinse-up procedure followed by purification on a silica gel column using CH₃OH/CH₂Cl₂ (1/1) as eluent 13.2 g oil was isolated. Identified as a cis/trans mixture of 3-hydroxymethyl-1-methyl-4-(3-trifluoromethylphenyl)piperidine by means of ¹H-NMR.

The compound was dissolved in toluene (300 ml), triethylamine (13.5 ml) was added, and the mixture stirred for 1 h. Subsequently benzenesulphonyl chloride (7.5 ml) was added, and the mixture stirred at RT for 70 h. The toluene phase was extracted with H₂O; the separated aqueous layer was extracted with ether and the combined ether and toluene phases were dried with MgSO₄, filtered and evaporated to dryness giving 10.3 g of an oil.

5 g of the oil, which was identified as 1-methyl-3-phenylsulphonyloxymethyl-4-(3-trifluoromethyl)piperidine by ¹H-NMR, was subsequently dissolved in MIBC (50 ml) and added to a solution of sesamol (1.9) and NaOH (0.5 g) in MIBC (150 ml). The mixture was refluxed for 2 h, stirred at RT overnight and extracted with H₂O. The MIBC-phase was evaporated to dryness, the residue was ecxtracted with NaOH/ether, the ether layer separated acetone and conc. HC1 (2 ml) was added resulting in a precipitate.

This was purified on a silica gel column using CHCl₂/CH₃OH 9/1 as solvent, yielding 1.1 g of (+-)-trans-1-methyl-3-(3,4-methylenedioxyphenoxymethyl)-4-(3-trifluoromethylphenyl)piperididne. M.p. 93.5°C
and
0.1 g of (+-)-cis-1-methyl-3-(3,4-methylene dioxyphenoxymethyl-4-(3-trifluoromethylphenyl)piperidine isolated as the oxalate identified by its ¹H-NMR and mass spectrum.

(+-)-3-(4-allyl-2-methoxyphenoxymethyl)-1-methyl-4-(3-trifluoromethylphenyl)piperidine oxalate was prepared from 1-methyl-3-phenylsulphonyloxymethyl-4-(3-trifluoromethyl)piperidine and eugenol as described above by reflux for 1.5 h. M.p. 43.5°C.

### EXAMPLE 8

### (+-)-trans-3-(3,4-methylenedioxyphenoxymethyl)-1-pentyl-4-(4-pentyloxyphenyl)piperidine hydrochloride

was prepared by refluxing 4-(4-hydroxyphenyl)-3-(3,4-methylenedioxyphenoxymethyl)piperidine )piperidine hydrochloride (0.35 g) with 1-bromopentane (1.8 ml) and K₂CO₃ (1 g) in abs. ethanol (25 ml) for 2 h. The rinse-up procedure described in example 1 gave the title compound. M.p. 148.2°C.

### EXAMPLE 9

### (+-)-3-(4-allyl-2-methoxyphenoxymethyl)-1-methyl-4-(4-trifluoromethylphenyl)piperidine oxalate

This compound was prepared by exactly the same reaction sequence as described in example 7 using arecolin and 4-bromotrifluorobenzene as the starting materials. The intermediates were identified by means of ¹H-NMR and so was the identity of the product confirmed.

### EXAMPLE 10

### (-)-trans-4-(4-fluorophenyl)-3-(2-iodo-4,5-methylenedioxyphenoxymethyl)-1-pentylpiperidine oxalate

(-)-trans-4-(4-fluorophenyl)-3-(3,4-methylenedioxyphenoxymethyl)-1-pentylpiperidine (1.2 g) was dissolved in CH₂Cl₂ (50 ml). Silver trifluoroacetate (0.66 g) was added, followed by iodine (0.76 g) in CH₂Cl₂ added over a 10 min. period. Stirring for 24 h at R.T. The mixture was filtered, extracted with OH⁻, the CH₂Cl₂-phase dried (NaSO₄) and subsequently evaporated to dryness. The residue was purified on silica gel and precipitated as the oxalate in acetone solution. M.p. 93.6-94.0°C.

### EXAMPLE 11

### (+-)-trans-3-(4-propenyl-2-methoxyphenoxymethyl)-4-(4-fluorophenyl)-1-pentylpiperidine oxalate

3-chloromethyl-4-(4-fluorophenyl)-1-pentylpiperidine (1 g) dissolved in dry DMF was added to a solution of eugenol (0.6 g) and sodium (0.09 g) in abs. ethanol 50 ml. The mixture heated to 100°C for 5 days. After 4 days NaH was added.

The reaction mixture was extracted with OH⁻ /ether, the etheral layer was dried (MgSO₄), evaporated to dryness and purified on a silica gel column using CH₂Cl₂/CH₃OH as eluent. Precipitated as the oxalate from acetone solution. Identified by ¹H and ¹³C NMR. M.p. 128.0-128.4°C.

## Claims (Claims for the following Contracting State(s): AT, BE, CH, LI, DE, FR, GB, GR, IT, LU, NL, SE)

1. Piperidine compounds selected from compounds having the general formula I 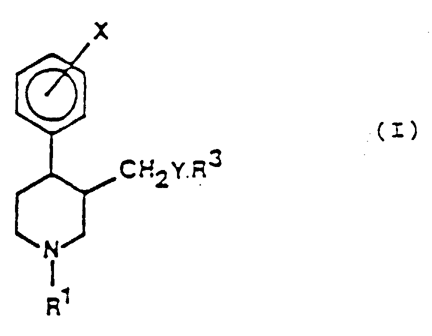 wherein
R³ is 3,4-methylenedioxyphenyl or phenyl which are optionally substituted with one or more halogen, C₁₋₆-alkoxy, C₁₋₆-alkyl, alkenyl having up to 6 carbon atoms or trifluoromethyl groups,
R¹ is straight or branched C₁₋₈-alkyl unsubstituted or substituted with one or more cyano, hydroxy, amido, halogeno, piperidino, morpholino, thiomorpholino, dioxolanyl, tetrahydrofuranyl, C₁₋₈-alkoxy or C₃₋₈ cycloalkyl groups,
X is hydrogen, halogen, trifluoromethyl, cyano or
C₁₋₈-alkoxy,
Y is O or S,
a salt thereof with a pharmaceutically acceptable acid, and compounds
(+ -)-trans-1-methyl-3-(6-bromo-2-naphthoxymethyl)-4-phenylpiperidine hydrochloride and
(+ -)-trams-3-(4-chloro-1-naphthoxymethyl)-1-methyl-4-phenylpiperidine oxalate
provided that R¹ is not unsubstituted C₁₋₈-alkyl, when R³ is 3,4-methylenedioxyphenyl or phenyl optionally substituted with one or more C₁₋₆-alkyl or C₁₋₆-alkoxy, and at the same time X is hydrogen or halogen, and
further provided that the following compounds are excluded:
cis-3-((4-methoxyphenoxy)-methyl)-1-methyl-4-(4-methoxyphenyl)-piperidine hydrochloride,
cis-3-phenoxymethyl-1-methyl-4-(4-methoxyphenyl)-piperidine hydrochloride,
(-)-cis-3-((4-methoxyphenoxy)-methyl)-1-trifluoroethyl-4-phenylpiperidine dec. hydrochloride,
(+)-cis-3-((4-methoxyphenoxy)-methyl)-1-trifluoroethyl-4-phenylpiperidine dec. hydrochloride,
(+-)-cis-3-((4-methoxyphenoxy)methyl)-1-trifluoroethyl-4-phenylpiperidine hydrobromide,
(-)trans-4-(4'-fluorophenyl)-1-methyl-3-(3'-trifluoromethylphenoxymethyl)-piperidine,
(±)trans-3-(4'-fluorophenoxymethyl)-4-(4'-fluorophenyl)-1-methylpiperidine and
α-3-((4-chlorophenoxy)-methyl)-1-methyl-4-phenyl-piperidine hydrobromide.

2. A compound of claim 1 wherein R³ is 3,4-methylenedioxy- phenyl, optionally substituted with halogen or C₁₋₆-alkoxy.

3. A compound of claim 1 wherein R¹ is straight or branched C₁₋₈-alkyl.

4. A compound of claim 1 wherein X is hydrogen, halogen trifluoromethyl or C₁₋₆-alkoxy.

5. A compound of claim 1 which is (-)-trans-3-(2-bromo-4,5-methylenedioxyphenoxymethyl)-1-butyl-4-(4-fluorophenyl)-piperidine hydrochloride.

6. A compound of claim 1 which is (-)-trans-4-(4-methoxyphenyl)-3-(3,4-methylenedioxyphenoxymethyl)-1-pentylpiperidine oxalate.

7. A compound of claim 1 which is (+)-trans-4-(4-methoxyphenyl)-3-(3,4-methylenedioxyphenoxymethyl)-1-pentyl-piperidine oxalate.

8. A compound of claim 1 which is (-)-trans-4-(-4-fluorophenyl)-3-(3,4-methylenedioxyphenoxymethyl)-1-(3-thiamorpholinylpropyl)-piperidine dihydrochloride.

9. A method of preparing a compound according to claim 1, comprising the following steps:
a) reacting a compound having the general formula II 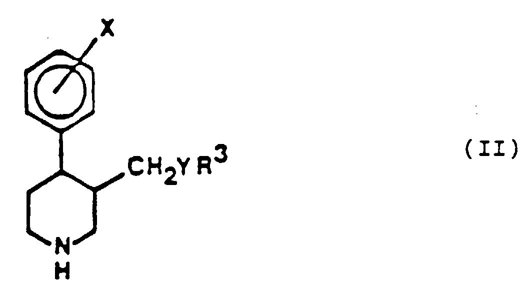 wherein R³, X and Y have the meanings defined above, with a compound having the general formula R¹-Z, wherein Z is a leaving group such as halogen and R¹ has the meaning defined above, or
b) reacting a compound having the general formula III 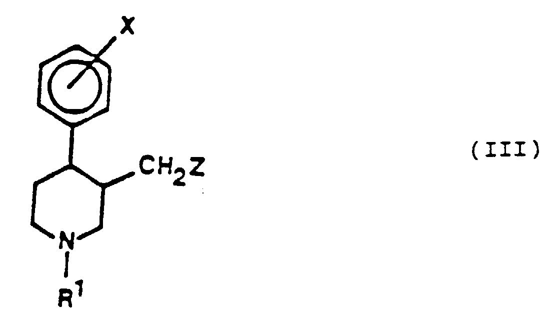 wherein R¹ and X have the meanings defined above, and Z is a leaving group, with a compound having the general formula R³-YH, wherein Y is O or S and R³ has the meaning defined above, or
c) reacting a compound having the general formula I, 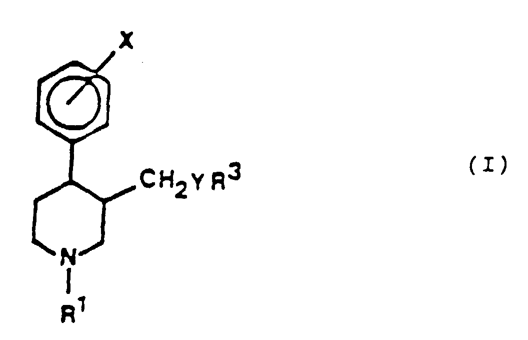 wherein X, R¹, R³ and Y have the meanings defined above, with bromine.

10. A pharmaceutical composition comprising a compound according to any of claims 1 to 8.

11. A pharmaceutical composition according to claim 10 wherein it is in the form of an oral dosage unit containing 1-100 mg of the active compound.

12. A compound of claim 1 for use as a therapeutic agent.

13. The use of a compound selected from compounds having the general formula I 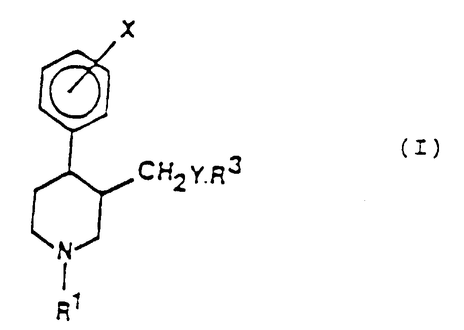 wherein
R³ is 3,4-methylenedioxyphenyl or phenyl which are optionally substituted with one or more halogen, C₁₋₆-alkoxy, C₁₋₆-alkyl, alkenyl having up to 6 carbon atoms or trifluoromethyl groups,
R¹ is straight or branched C₁₋₈-alkyl unsubstituted or substituted with one or more cyano, hydroxy, amido, halogeno, piperidino, morpholino, thiomorpholino, dioxolanyl, tetrahydrofuranyl, C₁₋₈-alkoxy or C₃₋₈ cycloalkyl groups,
X is hydrogen, halogen, trifluoromethyl, cyano or C₁₋₈-alkoxy,
Y is O or S
a salt thereof with a pharmaceutically acceptable acid, and compounds
(+ -)-trans-1-methyl-3-(6-bromo-2-naphthoxymethyl)-4-phenylpiperidine hydrochloride and
(+ -)-trans-3-(4-chloro-1-naphthoxymethyl)-1-methyl-4-phenylpiperidine oxalate
provided that R¹ is not unsubstituted C₁₋₈-alkyl, when R³ is 3,4-methylenedioxyphenyl or phenyl optionally substituted with one or more C₁₋₆-alkyl or C₁₋₆-alkoxy, and at the same time X is hydrogen or halogen,
for the preparation of a medicament useful in the treatment of calcium overload in brain cells of mammals.

## Claims (Claims for the following Contracting State(s): ES)

1. A method of preparing a piperidine compound selected from compounds having the general formula I 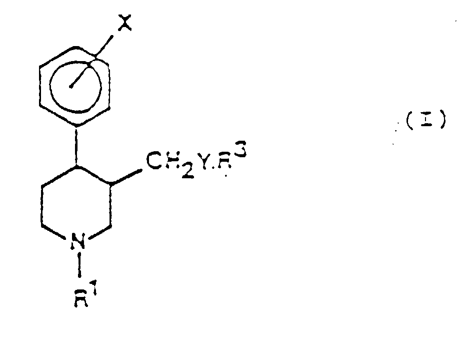 wherein
R³ is 3,4-methylenedioxyphenyl or phenyl which are optionally substituted with one or more halogen, C₁₋₆-alkoxy, C₁₋₆-alkyl, alkenyl having up to 6 carbon atoms or trifluoromethyl groups,
R¹ is straight or branched C₁₋₈-alkyl unsubstituted or substituted with one or more cyano, hydroxy, amido, halogeno, piperidino, morpholino, thiomorpholino, dioxolanyl, tetrahydrofuranyl, C₁₋₈-alkoxy or C₃₋₈-cycloalkyl groups,
X is hydrogen, halogen, trifluoromethyl, cyano or C₁₋₈-alkoxy,
Y is O or S,
a salt thereof with a pharmaceutically acceptable acid, and compounds
(+ -)-trans-1-methyl-3-(6-bromo-2-naphthoxymethyl)-4-phenylpiperidine hydrochloride and
(+ -)-trans-3-(4-chloro-1-naphthoxymethyl)-1-methyl-4-phenylpiperidine oxalate
provided that R¹ is not unsubstituted C₁₋₈-alkyl, when R³ is 3,4-methylenedioxyphenyl or phenyl optionally substituted with one or more C₁₋₆-alkyl or C₁₋₆-alkoxy, and at the same time X is hydrogen or halogen,
comprising the following steps:
a) reacting a compound having the general formula II 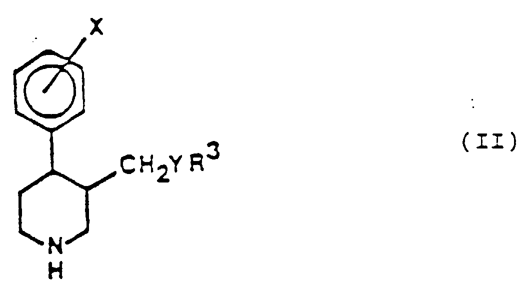 wherein R³, X and Y have the meanings defined above, with a compound having the general formula R¹-Z, wherein Z is a leaving group such as halogen and R¹ has the meaning defined above, or
b) reacting a compound having the general formula III 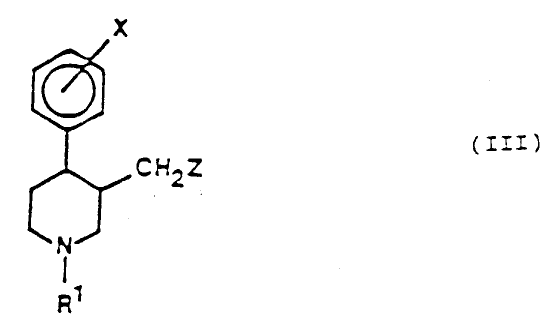 wherein R¹ and X have the meanings defined above, and Z is a leaving group, with a compound having the general formula R³-YH, wherein Y is O or S and R³ has the meaning defined above, or
c) reacting a compound having the general formula I, 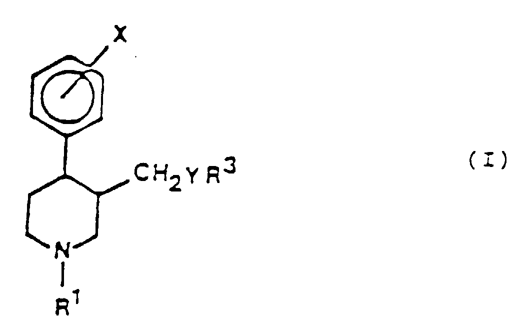 wherein X, R¹, R³ and Y have the meanings defined above, with bromine.

2. The method of claim 1 wherein R³ is 3,4-methylenedioxy-phenyl, optionally substituted with halogen or C₁₋₆-alkoxy.

3. The method of claim 1 wherein R¹ is straight or branched C₁₋₈-alkyl.

4. The method of claim 1 wherein X is hydrogen, halogen, trifluoromethyl or C₁₋₆-alkoxy.

5. The method of claim 1 wherein the compound is (-)-trans-3-(2-bromo-4,5-methylenedioxyphenoxymethyl)-1-butyl-4-(4-fluorophenyl)-piperidine hydrochloride.

6. The method of claim 1 wherein the compound is (-)-trans-4-(4-methoxy-phenyl)-3-(3,4-methylenedioxyphenoxymethyl)-1-pentyl-piperidine oxalate.

7. The method of claim 1 wherein the compound is (+)-trans-4-(4-methoxy-phenyl)-3-(3,4-methylenedioxyphenoxymethyl)-1-pentyl-piperidine oxalate.

8. The method of claim 1 wherein the compound is (-)-trans-4-(-4-fluoro-phenyl)-3-(3,4-methylenedioxyphenoxymethyl)-1-(3-thiamorpholinylpropyl)-piperidine dihydrochloride.

9. A method of preparing a pharmaceutical composition comprising a compound prepared according to any of claims 1 to 8, comprising the step of placing same together with a conventional adjuvant, carrier or diluent, optionally in the form of a pharmaceutically-acceptable acid addition salt thereof, into the form of a pharmaceutical composition.

10. A method according to claim 9 wherein the pharmaceutical composition is in the form of an oral dosage unit containing 1-100 mg of the active compound.

11. A method of preparing a medicament useful in the treatment of calcium overload in brain cells of mammals, characterised in the use, as an essential constituent of said medicament, of a compound selected from compounds having the general formula I 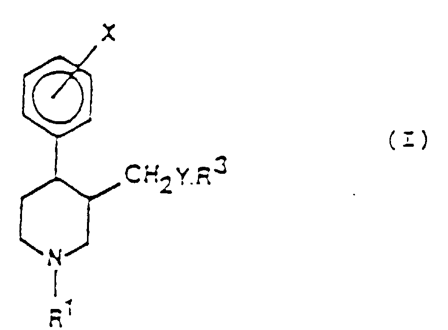 wherein
R³ is 3,4-methylenedioxyphenyl or phenyl which are optionally substituted with one or more halogen, C₁₋₆-alkoxy, C₁₋₆-alkyl, alkenyl having up to 6 carbon atoms or trifluoromethyl groups,
R¹ is straight or branched C₁₋₈-alkyl unsubstituted or substituted with one or more cyano, hydroxy, amido, halogeno, piperidino, morpholino, thiomorpholino, dioxolanyl, tetrahydrofuranyl, C₁₋₈-alkoxy or C₃₋₈ cycloalkyl groups,
X is hydrogen, halogen, trifluoromethyl, cyano or C₁₋₈-alkoxy,
Y is O or S
a salt thereof with a pharmaceutically acceptable acid, and compounds
(+ -)-trans-1-methyl-3-(6-bromo-2-naphthoxymethyl)-4-phenylpiperidine hydrochloride and
(+ -)-trans-3-(4-chloro-1-naphthoxymethyl)-1-methyl-4-phenylpiperidine oxalate
provided that R¹ is not unsubstituted C₁₋₈-alkyl, when R³ is 3,4-methylenedioxyphenyl or phenyl optionally substituted with one or more C₁₋₆-alkyl or C₁₋₆-alkoxy, and at the same time X is hydrogen or halogen.

## Patentansprüche (Patentansprüche für folgende(n) Vertragsstaat(en): AT, BE, CH, LI, DE, FR, GB, GR, IT, LU, NL, SE)

1. Piperidinverbindungen, ausgewählt aus Verbindungen mit der allgemeinen Formel I 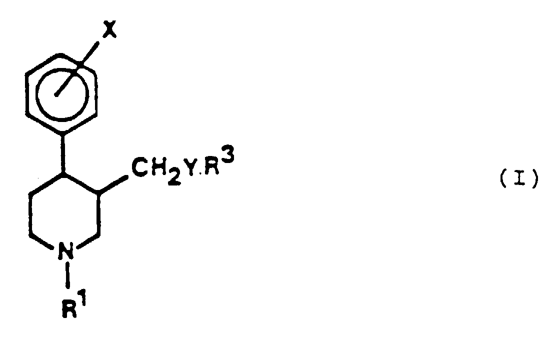 worin
R³ 3,4-Methylendioxyphenyl oder Phenyl bedeutet, welche gegebenenfalls mit einer oder mehreren Halogen-, C₁₋₆-Alkoxy-, C₁₋₆-Alkyl-, Alkenyl mit bis zu 6 Kohlenstoffatomen oder Tnfluormethyl-Gruppen substituiert sind,
R¹ ein geradkettiges oder verzweigtes C₁₋₈-Alkyl bedeutet, das unsubstituiert oder mit einer oder mehreren Cyano-, Hydroxy-, Amido-, Halogen-, Piperidin-, Morpholino-, Thiomorpholino-, Dioxolanyl-, Tetrahydrofuranyl-, C₁₋₈-Alkoxy- oder C₃₋₈-Cycloalkyl-Gruppen substituiert ist,
X Wasserstoff, Halogen, Trifluormethyl, Cyano oder C₁₋₈-Alkoxy bedeutet,
Y O oder S bedeutet,
einem Salz davon mit einer pharmazeutisch annehmbaren Säure, und den Verbindungen
(±)-trans-1-Methyl-3-(6-brom-2-naphthoxymethyl)4-phenylpiperidin-hydrochlorid und
(±)-trans-3-(4-Chlor-1-naphthoxymethyl)-1-methyl-4-phenylpiperidin-oxalat,
mit der Maßgabe, daß R¹ nicht unsubstituiertes C₁₋₈-Alkyl bedeutet, wenn R³ 3,4-Methylendioxyphenyl oder Phenyl bedeutet, das gegebenenfalls mit einem oder mehreren C₁₋₆-Alkyl- oder C₁₋₆-Alkoxy-Resten substituiert ist, und gleichzeitig X Wasserstoff oder Halogen bedeutet,
und
mit der weiteren Maßgabe, daß die folgenden Verbindungen ausgenommen sind:
cis-3-((4-Methoxyphenoxy)-methyl)-1-methyl-4-(4-methoxyphenyl)-piperidin-hydrochlorid,
cis-3-Phenoxymethyl-1-methyl-4-(4-methoxyphenyl)-piperidin-hydrochlorid,
(-)cis-3-((4-Methoxyphenoxy)-methyl)-1-trifluorethyl-4-phenylpiperidinhydrochlorid,
(+)-cis-3-((4-Methoxyphenoxy)-methyl)-1-trifluorethyl-4-phenylpiperidinhydrochlorid,
(±)-cis-3-((4-Methoxyphenoxy)-methyl)-1-trifluorethyl-4-phenylpiperidin-hydrobromid,
(-)-trans-4-(4'-Fluorphenyl)-1-methyl-3-(3'-trifluormethylphenoxymethyl)-piperidin,
(±)-trans-3-(4'-Fluorphenoxymethyl)-4-(4'-fluorphenyl)-1-methylpiperidin und
α-3-((4-Chlorphenoxy)-methyl)-1-methyl-4-phenyl-piperidin-hydrobromid.

2. Verbindung nach Anspruch 1, worin R³ 3,4-Methylendioxyphenyl bedeutet, das gegebenenfalls mit Halogen oder C₁₋₆-Alkoxy substituiert ist.

3. Verbindung nach Anspruch 1, worin R¹ ein geradkettiges oder verzweigtes C₁₋₈-Alkyl bedeutet.

4. Verbindung nach Anspruch 1, worin X Wasserstoff, Halogen, Trifluormethyl oder C₁₋₆-Alkoxy bedeutet.

5. Verbindung nach Anspruch 1, welche (-)-trans-3-(2-Brom-4,5-methylendioxyphenoxymethyl)-1-butyl-4-(4-fluorphenyl)-piperidin-hydrochlorid ist.

6. Verbindung nach Anspruch 1, welche (-)-trans-4-(4-Methoxyphenyl)-3-(3,4-methylendioxyphenoxymethyl)-1-pentyl-piperidin-oxalat ist.

7. Verbindung nach Anspruch 1, welche (+)-trans-4-(4-Methoxyphenyl)-3-(3,4-methylendioxyphenoxymethyl)-1-pentyl-piperidin-oxalat ist.

8. Verbindung nach Anspruch 1, welche (-)-trans-4-(4-Fluor-phenyl)-3-(3,4-methylendioxyphenoxymethyl)-1-(3-thiamorpholinylpropyl)-piperidin-dihydrochlorid ist.

9. Verfahren zum Herstellen einer Verbindung nach Anspruch 1, umfassend die folgenden Schritte:
a) Umsetzen einer Verbindung mit der allgemeinen Formel II 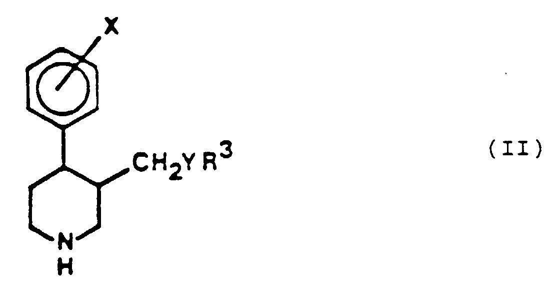 worin R³, X und Y die vorstehend angegebenen Bedeutungen haben, mit einer Verbindung mit der allgemeinen Formel R¹-Z, worin Z eine Austrittsgruppe wie Halogen bedeutet und R¹ die vorstehend angegebene Bedeutung hat, oder
b) Umsetzen einer Verbindung mit der allgemeinen Formel III 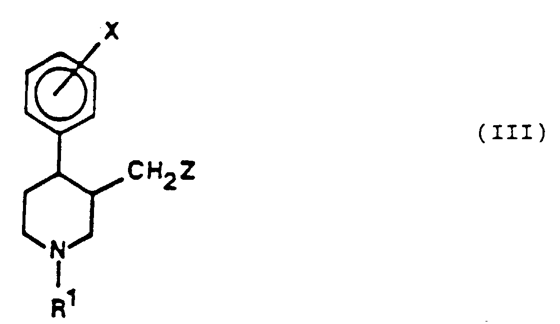 worin R¹ und X die vorstehend angegebenen Bedeutungen haben, und Z eine Austrittsgruppe ist, mit einer Verbindung mit der allgemeinen Formel R³-YH, worin Y O oder S bedeutet und R³ die vorstehend angegebene Bedeutung hat, oder
c) Umsetzen einer Verbindung mit der allgemeinen Formel I 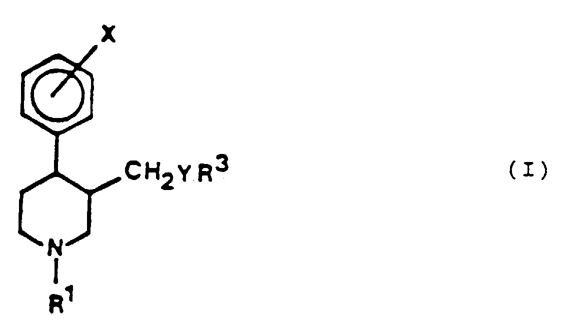 worin X, R¹, R³ und Y die vorstehend angegebenen Bedeutungen haben, mit Brom.

10. Pharmazeutische Zusammensetzung umfassend eine Verbindung nach einem der Ansprüche 1 bis 8.

11. Pharmazeutische Zusammensetzung nach Anspruch 10, welche in der Form einer oralen Dosiseinheit, die 1-100 mg der Wirkstoffverbindung enthält, vorliegt.

12. Verbindung nach Anspruch 1 zur Verwendung als Heilmittel.

13. Verwendung einer Verbindung, ausgewählt aus Verbindungen mit der allgemeinen Formel I 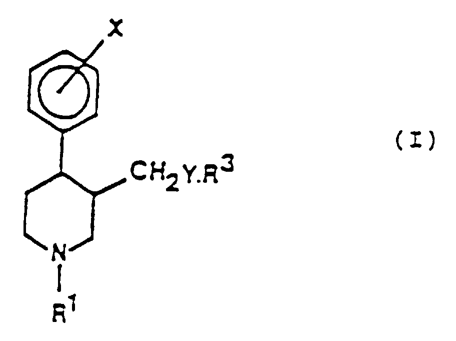 worin
R³ 3,4-Methylendioxyphenyl oder Phenyl bedeutet, welche gegebenenfalls mit einer oder mehreren Halogen-, C₁₋₆-Alkoxy-, C₁₋₆-Alkyl-, Alkenyl mit bis zu 6 Kohlenstoffatomen oder Trifluormethyl-Gruppen substituiert sind,
R¹ ein geradkettiges oder verzweigtes C₁₋₈-Alkyl bedeutet, das unsubstituiert oder mit einer oder mehreren Cyano-, Hydroxy-, Amido-, Halogen-, Piperidin-, Morpholino-, Thiomorpholino-, Dioxolanyl-, Tetrahydrofuranyl-, C₁₋₈-Alkoxy- oder C₃₋₈-Cycloalkyl-Gruppen substituiert ist,
X Wasserstoff, Halogen, Trifluormethyl, Cyano oder C₁₋₈-Alkoxy bedeutet,
Y O oder S bedeutet,
einem Salz davon mit einer pharmazeutisch annehmbaren Säure, und den Verbindungen
(±)-trans-1 -Methyl-3-(6-brom-2-naphthoxymethyl)-4-phenylpiperidin-hydrochlorid und
(±)-trans-3-(4-Chlor-1-naphthoxymethyl)-1-methyl-4-phenylpiperidin-oxalat
mit der Maßgabe, daß R¹ nicht unsubstituiertes C₁₋₈-Alkyl bedeutet, wenn R³ 3,4-Methylendioxyphenyl oder Phenyl bedeutet, das gegebenenfalls mit einem oder mehreren C₁₋₆-Alkyl- oder C₁₋₆-Alkoxy-Resten substituiert ist, und gleichzeitig X Wasserstoff oder Halogen bedeutet,
zur Herstellung eines Arzneimittels, das bei der Behandlung eines überhöhten Calciumgehalts in Gehimzellen von Säugetieren brauchbar ist.

## Patentansprüche (Patentansprüche für folgende(n) Vertragsstaat(en): ES)

1. Verfahren zum Herstellen einer Piperidinverbindung, ausgewählt aus Verbindungen mit der allgemeinen Formel I 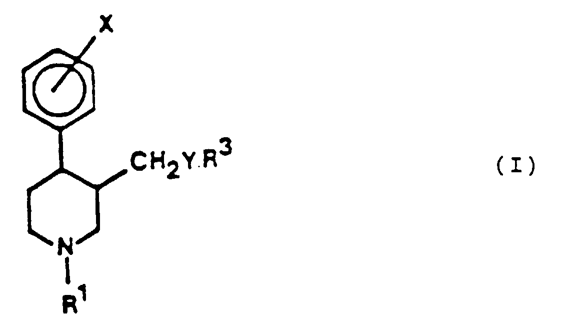 worin
R³ 3,4-Methylendioxyphenyl oder Phenyl bedeutet, welche gegebenenfalls mit einer oder mehreren Halogen-, C₁₋₆-Alkoxy-, C₁₋₆-Alkyl-, Alkenyl mit bis zu 6 Kohlenstoffatomen oder Trifluormethyl-Gruppen substituiert sind,
R¹ ein geradkettiges oder verzweigtes C₁₋₈-Alkyl bedeutet, das unsubstituiert oder mit einer oder mehreren Cyano-, Hydroxy-, Amido-, Halogen-, Piperidin-, Morpholino-, Thiomorpholino-, Dioxolanyl-, Tetrahydrofuranyl-, C₁₋₈-Alkoxy- oder C₃₋₈-Cycloalkyl-Gruppen substituiert ist,
X Wasserstoff, Halogen, Trifluormethyl, Cyano oder C₁₋₈-Alkoxy bedeutet,
Y O oder S bedeutet,
einem Salz davon mit einer pharmazeutisch annehmbaren Säure, und den Verbindungen
(±)-trans-1-Methyl-3-(6-brom-2-naphthoxymethyl)-4-phenylpiperidin-hydrochlorid und
(±)-trans-3-(4-Chlor-1-naphthoxymethyl)-1-methyl-4-phenylpiperidin-oxalat
mit der Maßgabe, daß R¹ nicht unsubstituiertes C₁₋₈-Alkyl bedeutet, wenn R³ 3,4-Methylendioxyphenyl oder Phenyl bedeutet, das gegebenenfalls mit einem oder mehreren C₁₋₆-Alkyl- oder C₁₋₆-Alkoxy-Resten substituiert ist, und gleichzeitig X Wasserstoff oder Halogen ist,
umfassend die folgenden Schritte:
a) Umsetzen einer Verbindung mit der allgemeinen Formel II 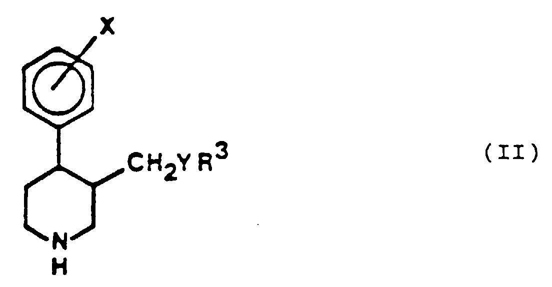 worin R³, X und Y die vorstehend angegebenen Bedeutungen haben, mit einer Verbindung mit der allgemeinen Formel R¹-Z, worin Z eine Austrittsgruppe wie Halogen ist und R¹ die vorstehend angegebene Bedeutung hat, oder
b) Umsetzen einer Verbindung mit der allgemeinen Formel III 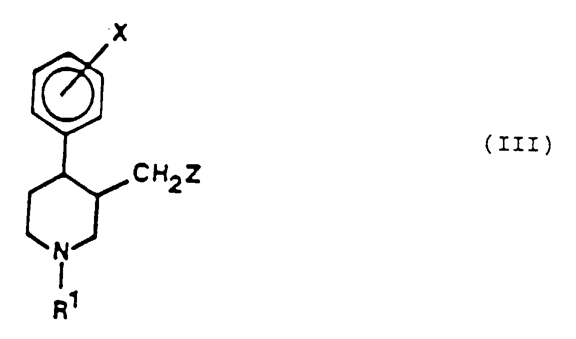 worin R¹ und X die vorstehend angegebenen Bedeutungen haben, und Z eine Austrittsgruppe ist, mit einer Verbindung mit der allgemeinen Formel R³-YH, worin Y O oder S ist und R³ die vorstehend angegebene Bedeutung hat, oder
c) Umsetzen einer Verbindung mit der allgemeinen Formel I 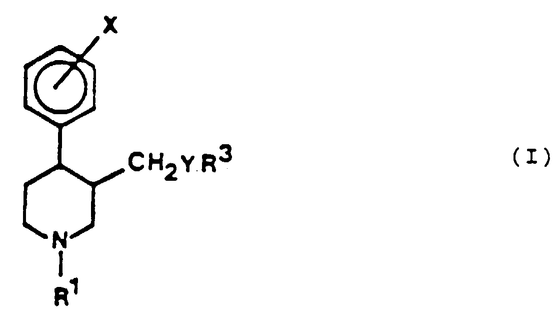 worin X, R¹, R³ und Y die vorstehend angegebenen Bedeutungen haben, mit Brom.

2. Verfahren nach Anspruch 1, worin R³ 3,4-Methylendioxyphenyl, das gegebenenfalls mit Halogen oder C₁₋₆-Alkoxy substituiert ist, bedeutet.

3. Verfahren nach Anspruch 1, worin R¹ ein geradkettiges oder verzweigtes C₁₋₈-Alkyl bedeutet.

4. Verfahren nach Anspruch 1, worin X Wasserstoff, Halogen, Trifluormethyl oder C₁₋₆-Alkoxy bedeutet.

5. Verfahren nach Anspruch 1, worin die Verbindung (-)-trans-3-(2-Brom-4,5-methylendioxyphenoxymethyl)-1-butyl-4-(4-fluorphenyl)-piperidin-hydrochlorid ist.

6. Verfahren nach Anspruch 1, worin die Verbindung (-)-trans-4-(4-Methoxyphenyl)-3-(3,4-methylendioxyphenoxymethyl)-1-pentyl-piperidin-oxalat ist.

7. Verfahren nach Anspruch 1, worin die Verbindung (+)-trans-4-(4-Methoxyphenyl)-3-(3,4-methylendioxyphenoxymethyl)-1-pentyl-piperidin-oxalat ist.

8. Verfahren nach Anspruch 1, worin die Verbindung (-)-trans-4-(4-Fluorphenyl)-3-(3,4-methylendioxyphenoxymethyl)-1-(3-thiamorpholinylpropyl)-piperidin-dihydrochlorid ist.

9. Verfahren zum Herstellen einer pharmazeutischen Zusammensetzung umfassend eine Verbindung, die nach einem der Ansprüche 1 bis 8 hergestellt ist, umfassend den Schritt, daß man diese Verbindung zusammen mit einem herkömmlichen Adjuvans, Träger oder Verdünnungsmittel, gegebenenfalls in Form eines pharmazeutisch annehmbaren Säureadditionssalzes davon, in die Form einer pharmazeutischen Zusammensetzung bringt.

10. Verfahren nach Anspruch 9, worin die pharmazeutische Zusammensetzung in Form einer oralen Dosiseinheit, welche 1-100 mg der Wirkstoffverbindung enthält, vorliegt.

11. Verfahren zum Herstellen eines Arzneimittels, das bei der Behandlung eines überhöhten Calciumgehalts in Gehimzellen von Säugetieren brauchbar ist, **gekennzeichnet durch** die Verwendung einer Verbindung, ausgewählt aus Verbindungen mit der allgemeinen Formel I 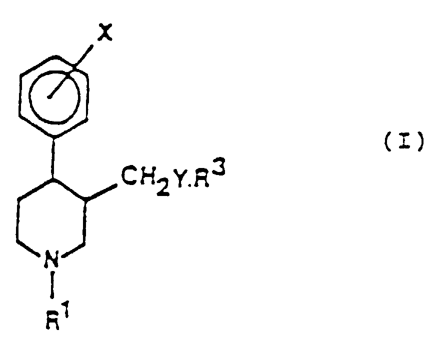 worin
R³ 3,4-Methylendioxyphenyl oder Phenyl bedeutet, welche gegebenenfalls mit einer oder mehreren Halogen-, C₁₋₆-Alkoxy-, C₁₋₆-Alkyl-, Alkenyl mit bis zu 6 Kohlenstoffatomen oder Trifluormethyl-Gruppen substituiert sind,
R¹ ein geradkettiges oder verzweigtes C₁₋₈-Alkyl bedeutet, das unsubstituiert oder mit einer oder mehreren Cyano-, Hydroxy-, Amido-, Halogen-, Piperidin-, Morpholino-, Thiomorpholino-, Dioxolanyl-, Tetrahydrofuranyl-, C₁₋₈-Alkoxy- oder C₃₋₈-Cycloalkyl-Gruppen substituiert ist,
X Wasserstoff, Halogen, Trifluormethyl, Cyano oder C₁₋₈-Alkoxy bedeutet,
Y O oder S bedeutet,
einem Salz davon mit einer pharmazeutisch annehmbaren Säure, und den Verbindungen
(±)-trans-1-Methyl-3-(6-brom-2-naphthoxymethyl)-4-phenylpiperidin-hydrochlorid und
(±)-trans-3-(4-Chlor-1-naphthoxymethyl)-1-methyl-4-phenylpiperidin-oxalat
mit der Maßgabe, daß R¹ nicht unsubstituiertes C₁₋₈-Alkyl bedeutet, wenn R³ 3,4-Methylendioxyphenyl oder Phenyl bedeutet, das gegebenenfalls mit einem oder mehreren C₁₋₆-Alkyl- oder C₁₋₆-Alkoxy-Resten substituiert ist, und gleichzeitig X Wasserstoff oder Halogen bedeutet,
als einen wesentlichen Bestandteil des Arzneimittels.

## Revendications (Revendications pour l'(les) Etat(s) contractant(s) suivant(s): AT, BE, CH, LI, DE, FR, GB, GR, IT, LU, NL, SE)

1. Composé de pipéridine sélectionné à partir des composés ayant la formule générale I 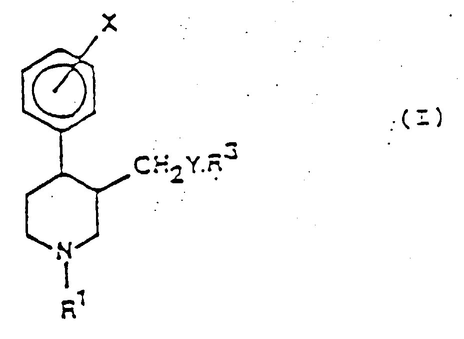 dans laquelle
R³ est un 3,4-méthylènedioxyphényle ou un phényle qui sont éventuellement substitués avec un ou plusieurs groupes halogène, alcoxy en C₁ à C₆, alkyle en C₁ à C₆, alcényle ayant jusqu'à 6 atomes de carbone, ou trifluorométhyle,
R¹ est un alkyle en C₁ à C₈, linéaire ou ramifié, non substitué ou substitué avec un ou plusieurs groupe(s) cyano, hydroxy, amido, halogéno, pipéridino, morpholino, thiomorpholino, dioxolanyle, tétrahydrofuranyle, alcoxy en C₁ à C₈ ou cycloalkyle en C₃ à C₈,
X est un hydrogène, un halogène, un trifluorométhyle, un cyano ou un alcoxy en C₁ à C₈,
Y est O ou S,
un sel de celui-ci avec un acide pharmaceutiquement acceptable et des composés de chlorhydrate de (+-)-trans-1-méthyl-3-(6-bromo-2-naphthoxyméthyl)-4-phénylpipéridine et d'oxalate de (+-)-trans-3-(4-chloro-1-naphthoxyméthyl)-1-méthyl-4-phénylpipéridine
pourvu que R¹ ne soit pas un alkyle en C₁ à C₈ non substitué, lorsque R³ est un 3,4-méthylènedioxyphényle ou un phényle éventuellement substitué avec un ou plusieurs alkyle en C₁ à C₆ ou alcoxy en C₁ à C₆, et que X est un hydrogène ou un halogène, et
pourvu encore que les composés suivants soient exclus :
chlorhydrate de cis-3-((4-méthoxyphénoxy)-méthyl)-1-méthyl-4-(4-méthoxyphényl)-pipéridine,
chlorhydrate de cis-3-phénoxyméthyl-1-méthyl-4-(4-méthoxyphényl)-pipéridine,
dec. chlorhydrate de (-)-cis-3-((4-méthoxyphénoxy)-méthyl)-1-trifluoroéthyl-4-phénylpipéridine,
dec. chlorhydrate de (+)-cis-3-((4-méthoxyphénoxy)-méthyl)-1-trifluoroéthyl-4-phénylpipéridine,
bromhydrate de (+-)-cis-3-((4-méthoxyphénoxy)méthyl)-1-trifluoroéthyl-4-phénylpipéridine,
(-)trans-4-(4'-fluorophényl)-1-méthyl-3-(3'-trifluorométhylphénoxyméthyl)-pipéridine,
(±)trans-3-(4'-fluorophénoxyméthyl)-4-(4'-fluorophényl)-1-méthylpipéridine et
bromhydrate de α-3-((4-chlorophénoxy)-méthyl)-1-méthyl-4-phényl- pipéridine.

2. Composé de la revendication 1, dans lequel R³ est un 3,4-méthylènedioxyphényle, éventuellement substitué avec un halogène ou un alcoxy en C₁ à C₆.

3. Composé de la revendication 1, dans lequel R¹ est un alkyle en C₁ à C₈, linéaire ou ramifié.

4. Composé de la revendication 1, dans lequel X est un hydrogène, un halogène, un trifluorométhyl ou un alcoxy en C₁ à C₆.

5. Composé de la revendication 1, qui est un chlorhydrate de (-)-trans-3-(2-bromo-4,5-méthylènedioxyphénoxyméthyl)-1-butyl-4-(4-fluorophényl)pipéridine.

6. Composé de la revendication 1, qui est un oxalate de (-)-trans-4-(4-méthoxy-phényl)-3-(3,4-méthylènedioxyphénoxyméthyl)-1-pentyl-pipéridine.

7. Composé de la revendication 1 qui est un oxalate de (+)-trans-4-(4-méthoxy-phényl)-3-(3,4-méthylènedioxyphénoxyméthyl)-1-pentyl-pipéridine.

8. Composé de la revendication 1 qui est un dichlorhydrate de (-)-trans-4-(4-fluorophényl)-3-(3,4-méthylènedioxyphénoxyméthyl)-1-(3-thiamorpholinylpropyl)pipéridine.

9. Procédé de préparation d'un composé selon la revendication 1, comprenant les étapes suivantes :
a) faire réagir un composé ayant la formule générale II 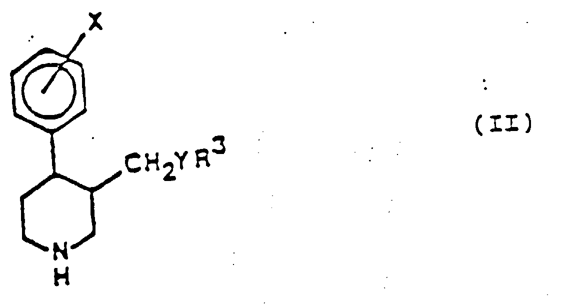 dans laquelle R³, X et Y ont les significations définies ci-dessus, avec un composé ayant la formule générale R¹-Z, dans laquelle Z est un groupe partant tel qu'un halogène et R¹ a la signification définie ci-dessus, ou
b) faire réagir un composé ayant la formule générale III 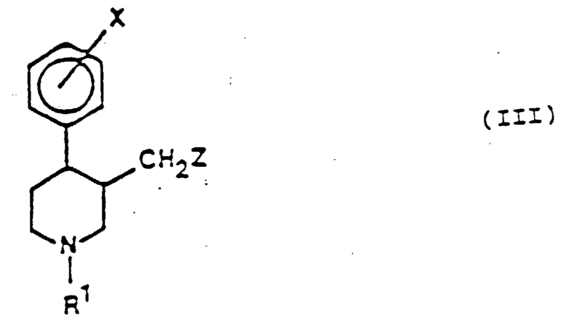 dans laquelle R¹ et X ont les significations définies ci-dessus, et Z est un groupe partant, avec un composé ayant la formule générale R³-YH, dans laquelle Y est O ou S et R³ a la signification définie ci-dessus, ou
c) faire réagir un composé ayant la formule générale I. 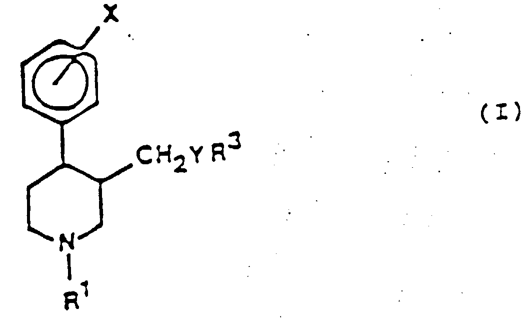 dans laquelle X, R¹, R³ et Y ont les significations définies ci-dessus, avec du brome.

10. Composition pharmaceutique comprenant un composé selon l'une quelconque des revendications 1 à 8.

11. Composition pharmaceutique selon la revendication 10 dans laquelle il est sous la forme d'une unité posologique administrée par voie orale contenant 1 à 100 mg du composé actif.

12. Composé de la revendication 1 pour une utilisation en tant qu'agent thérapeutique.

13. Utilisation d'un composé sélectionné à partir de composés ayant la formule générale I 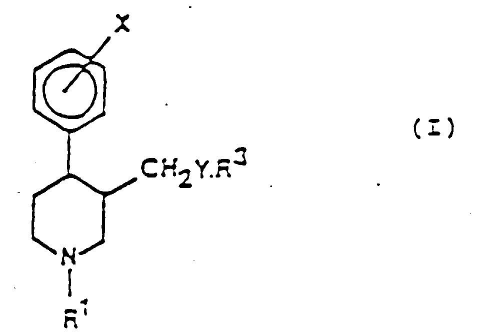 dans laquelle
R³ est un 3,4-méthylènedioxyphényle ou un phényle qui sont éventuellement substitués avec un ou plusieurs groupe(s) halogène, alcoxy en C₁ à C₆, alkyle en C₁ à C₆, alcényle ayant jusqu'à 6 atomes de carbone ou trifluorométhyle,
R¹ est un alkyle en C₁ à C₈, linéaire ou ramifié, non substitué ou substitué avec un ou plusieurs groupe(s) cyano, hydroxy, amido, halogéno, pipéridino, morpholino, thiomorpholino, dioxolanyle, tétrahydrofuranyle, alcoxy en C₁ à C₈ ou cycloalkyle en C₃ à C₈,
X est un hydrogène, un halogène, un trifluorométhyle, un cyano ou un alcoxy en C₁ à C₈,
Y est O ou S,
un sel de celui-ci avec un acide pharmaceutiquement acceptable et des composés de chlorhydrate de (+-)-trans-1-méthyl-3-(6-bromo-2-naphthoxyméthyl)-4-phénylpipéridine et d'oxalate de (+-)-trans-3-(4-chloro-1-naphthoxyméthyl)-1-méthyl-4-phénylpipéridine
pourvu que R¹ ne soit pas un alkyle en C₁ à C₈ non substitué, lorsque R³ est le 3,4-méthylènedioxyphényle ou le phényle éventuellement substitué avec un ou plusieurs alkyle en C₁ à C₆ ou alcoxy en C₁ à C₆, et que X est un hydrogène ou un halogène,
pour la préparation d'un médicament utilisable dans le traitement d'une surcharge en calcium dans des cellules cérébrales de mammifère.

## Revendications (Revendications pour l'(les) Etat(s) contractant(s) suivant(s): ES)

1. Procédé de préparation d'un composé de pipéridine sélectionné à partir des composés ayant la formule générale I 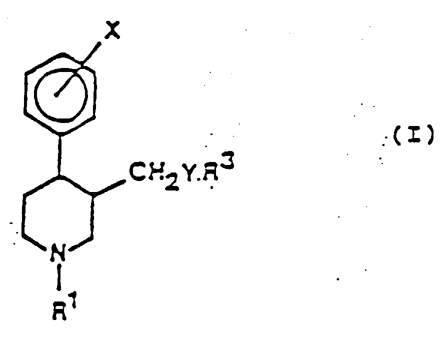 dans laquelle
R³ est un 3,4-méthylènedioxyphényle ou un phényle qui sont éventuellement substitués avec un ou plusieurs groupe(s) halogène, alcoxy en C₁ à C₆, alkyle en C₁ à C₆, alcényle ayant jusqu'à 6 atomes de carbone, ou trifluorométhyle,
R¹ est un alkyle en C₁ à C₈, linéaire ou ramifié, non substitué ou substitué avec un ou plusieurs groupe(s) cyano, hydroxy, amido, halogéno, pipéridino, morpholino, thiomorpholino, dioxolanyle, tétranydrofuranyle, alcoxy en C₁ à C₈ ou cycloalkyle en C₃ à C₈,
X est un hydrogène, un halogène, un trifluorométhyle, un cyano ou un alcoxy en C₁ à C₈,
Y est O ou S,
un sel de celui-ci avec un acide pharmaceutiquement acceptable et des composés de chlorhydrate de (+-)-trans-1-méthyl-3-(6-bromo-2-naphthoxyméthyl)-4-phénylpipéridine et d'oxalate de (+-)-trans-3-(4-chloro-1-naphthoxyméthyl)-1-méthyl-4-phénylpipéridine
pourvu que R¹ ne soit pas un alkyle en C₁ à C₈ non substitué, lorsque R³ est un 3,4-méthylènedioxyphényle ou un phényle éventuellement substitué avec un ou plusieurs alkyle en C₁ à C₆ ou alcoxy en C₁ à C₆, et que X est l'hydrogène ou un halogène,
comprenant les étapes suivantes :
a) faire réagir un composé ayant la formule générale II 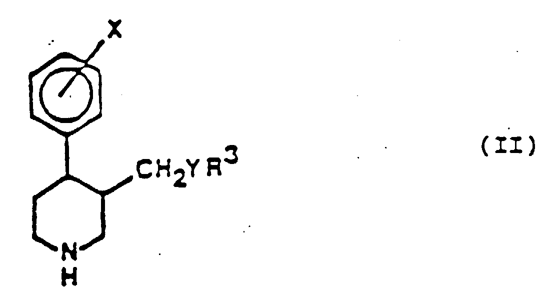 dans laquelle R³, X et Y ont les significations définies ci-dessus, avec un composé ayant la formule générale R¹-Z, dans laquelle Z est un groupe partant tel qu'un halogène et R¹ a la signification définie ci-dessus, ou
b) faire réagir un composé ayant la formule générale III 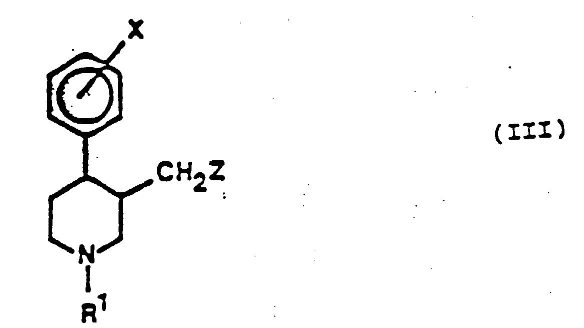 dans laquelle R¹ et X ont les significations définies ci-dessus, et Z est un groupe partant, avec un composé ayant la formule générale R³-YH, dans laquelle Y est O ou S et R³ a la signification définie ci-dessus, ou
c) faire réagir un composé ayant la formule générale I, 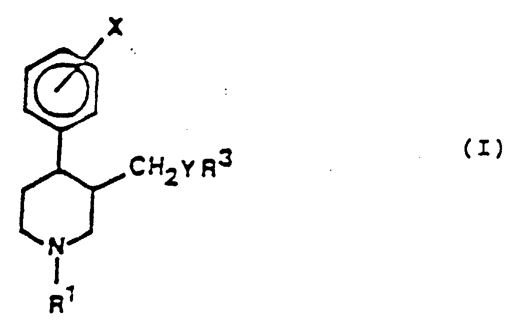 dans laquelle X, R¹, R³ et Y ont les significations définies ci-dessus, avec du brome.

2. Procédé de la revendication 1, dans lequel R³ est un 3,4-méthylènedioxyphényle, éventuellement substitué avec un halogène ou un alcoxy en C₁ à C₆.

3. Procédé de la revendication 1, dans lequel R¹ est un alkyle en C₁ à C₈, linéaire ou ramifié.

4. Procédé de la revendication 1, dans lequel X est l'hydrogène, un halogène, un trifluorométhyl ou un alcoxy en C₁ à C₆.

5. Procédé de la revendication 1, dans lequel le composé est un chlorhydrate de (-)-trans-3-(2-bromo-4,5-méthylènedioxyphénoxyméthyl)-1-butyl-4-(4-fluorophényl)pipéridine.

6. Procédé de la revendication 1, dans lequel le composé est un oxalate de (-)-trans-4-(4-méthoxyphényl)-3-(3,4-méthylènedioxyphénoxyméthyl)-1-pentyl-pipéridine.

7. Procédé de la revendication 1 dans lequel le composé est un oxalate de (+)-trans-4-(4-méthoxy-phényl)-3-(3,4-méthylènedioxyphénoxyméthyl)-1-pentyl-pipéridine.

8. Procédé de la revendication 1, dans lequel le composé est un dichlorhydrate de (-)-trans-4-(4-fluorophényl)-3-(3,4-méthylènedioxyphénoxyméthyl)-1-(3-thiamorpholinylpropyl)-pipéridine.

9. Procédé de préparation d'une composition pharmaceutique comprenant un composé préparé selon l'une quelconque des revendications 1 à 8, comprenant l'étape consistant à mettre celui-ci avec un adjuvant, excipient ou diluant classique, éventuellement sous la forme d'un sel d'addition acide pharmaceutiquement acceptable de celui-ci, sous la forme d'une composition pharmaceutique.

10. Procédé selon la revendication 9, dans lequel la composition pharmaceutique est sous la forme d'une unité posologique administrée par voie orale contenant 1 à 100 mg du composé actif.

11. Procédé de préparation d'un médicament utilisable dans le traitement d'une surcharge en calcium dans les cellules cérébrales de mammifères, caractérisé par l'utilisation, comme constituant essentiel dudit médicament, d'un composé sélectionné à partir de composés ayant la formule générale I 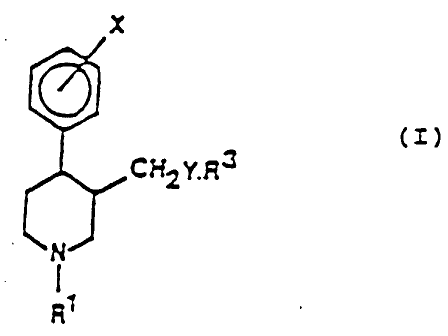 dans laquelle
R³ est le 3,4-méthylènedioxyphényle ou le phényle qui sont éventuellement substitués avec un ou plusieurs groupe(s) halogène, alcoxy en C₁ à C₆, alkyle en C₁ à C₆, alcényle ayant jusqu'à 6 atomes de carbone ou trifluorométhyle,
R¹ est un alkyle en C₁ à C₈, linéaire ou ramifié, non substitué ou substitué avec un ou plusieurs groupe(s) cyano, hydroxy, amido, halogéno, pipéridino, morpholino, thiomorpholino, dioxolanyle, tétrahydrofuranyle, alcoxy en C₁ à C₈ ou cycloalkyle en C₃ à C₈,
X est un hydrogène, un halogène, un trifluorométhyle, un cyano ou un alcoxy en C₁ à C₈,
Y est O ou S,
un sel de celui-ci avec un acide pharmaceutiquement acceptable et des composés de chlorhydrate de (+-)-trans-1-méthyl-3-(6-bromo-2-naphthoxyméthyl)-4-phénylpipéridine et d'oxalate de (+-)-trans-3-(4-chloro-1-naphthoxyméthyl)-1-méthyl-4-phénylpipéridine
pourvu que R¹ ne soit pas un alkyle en C₁ à C₈ non substitué, lorsque R³ est le 3,4-méthylènedioxyphényle ou le phényle éventuellement substitué avec un ou plusieurs alkyle en C₁ à C₆ ou alcoxy en C₁ à C₆, et que X est un hydrogène ou un halogène.
